**Europäisches Patentamt**

**European Patent Office**

**Office européen des brevets**

(19)

(11) Veröffentlichungsnummer: **0 047 378**
**B1**

(12) # EUROPÄISCHE PATENTSCHRIFT

(45) Veröffentlichungstag der Patentschrift:
29.05.85

(51) Int. Cl.⁴: **B 29 C 47/88**

(21) Anmeldenummer: **81105645.6**

(22) Anmeldetag: **18.07.81**

(54) **Verfahren zur Kühlung von Kunststoff-Hohlprofilen.**

(30) Priorität: **03.09.80 DE 3033054**

(43) Veröffentlichungstag der Anmeldung:
**17.03.82 Patentblatt 82/11**

(45) Bekanntmachung des Hinweises auf die Patenterteilung:
**29.05.85 Patentblatt 85/22**

(84) Benannte Vertragsstaaten:
**AT BE FR GB IT NL**

(56) Entgegenhaltungen:
**DE - A - 2 324 133**
**DE - A - 2 345 957**
**DE - A - 2 456 386**
**FR - A - 2 442 714**

(73) Patentinhaber: **MESSER GRIESHEIM GMBH, Hanauer Landstrasse 330, D-6000 Frankfurt/Main 1 (DE)**

(72) Erfinder: **v. Hoesslin, Gerhard, Kronenstrasse 66, D-4000 Düsseldorf (DE)**
Erfinder: **Hesse, Jürgen, Vogtswiese 60, D-7060 Schorndorf (DE)**

# Beschreibung

Die Erfindung betrifft ein Verfahren zur Kühlung von Kunststoff-Hohlprofilen beim Extrudieren durch direkten Wärmeaustausch mit flüssigem Stickstoff, der durch eine Bohrung im Dorn des Extrusionswerkzeuges in das Innere des Kunststoff-Hohlprofils eingeführt wird.

Die Innenkühlung von extrudierten Kunststoff-Hohlprofilen, z. B. Großrohren, mit flüssigem Stickstoff als Kühlmedium ist bekannt, z. B. aus der DE-A-2 324 133.

Bei kleinen Kunststoff-Hohlprofilen, z. B. Fensterprofilen aus Thermoplasten, ist wegen der kleinen Querschnitte eine derartige konventionelle Innenkühlung nicht möglich. Bei einer Außenkalibrierung und der Kühlung mit Wasser von außen ist die Abkühlgeschwindigkeit und damit die Extrusionsgeschwindigkeit begrenzt. Bei der Innenkühlung ist es dagegen ab bestimmter Größenverhältnisse nicht mehr möglich, genügend flüssigen Stickstoff durch den Dorn in das Hohlprofil zu leiten. Auch eine Isolation der Kühlmittelleitungen im Werkzeug, wie sie beispielsweise die FR-A-2 442 714 zeigt, würde nur eine verhältnismäßig geringfügige Verbesserung bringen.

Der Erfindung liegt die Aufgabe zugrunde, ein Verfahren zu schaffen, welches eine Innenkühlung von Kunststoff-Hohlprofilen mit flüssigem Stickstoff selbst bei solchen Profilen ermöglicht, bei denen bisher wegen ihrer geringen Größe diese Art der Kühlung ausgeschlossen schien.

Ausgehend von dem im Oberbegriff des Anspruches berücksichtigten Stand der Technik ist diese Aufgabe erfindungsgemäß gelöst mit den im kennzeichnenden Teil des Anspruches angegebenen Merkmalen.

Flüssiger Stickstoff ist als intensiv wirkendes Kühlmedium bekannt. Wegen der geringen Querschnitte der Hohlkammer-Werkzeuge erschien es jedoch nicht möglich, ihn für die Innenkühlung von Kunststoff-Hohlprofilen beim Extrudieren einzusetzen. Da diese Werkzeuge Temperaturen von 150 bis 180°C haben und aus konstruktiven Gründen die Bohrung im Extrusionswerkzeug für die Zufuhr des flüssigen Stickstoffs 3 mm nicht überschreiten darf, bei Bohrungslängen von 100 bis 150 mm, war zu erwarten, daß der flüssige Stickstoff beim Durchströmen des Werkzeuges verdampfen würde, so daß das Hohlprofil nur noch mit mehr oder weniger kaltem gasförmigen Stickstoff beaufschlagt würde. Durch den erfindungsgemäßen Einsatz von unterkühltem flüssigem Stickstoff in Verbindung mit einem besonders isolierten Kapillarschlauch wurde jedoch überraschenderweise erreicht, daß der Stickstoff weitgehend in flüssiger Form in das extrudierte Kunststoff-Hohlprofil gelangt.

Die Zeichnungen veranschaulichen ein Ausführungsbeispiel mit der Erfindung und eine Versuchseinrichtung, in der das erfindungsgemäße Verfahren unter verschiedenen Arbeitsbedingungen durchgeführt wurde. Es zeigt

Fig. 1 den Austrittsbereich eines Extrusionswerkzeuges im Schnitt,

Fig. 2 eine Versuchsanlage zur Prüfung des Verfahrens unter verschiedenen Arbeitsbedingungen,

Fig. 3 ein Detail der Versuchsanlage von Fig. 2,

Fig. 4 eine Draufsicht auf das Detail gemäß Fig. 3.

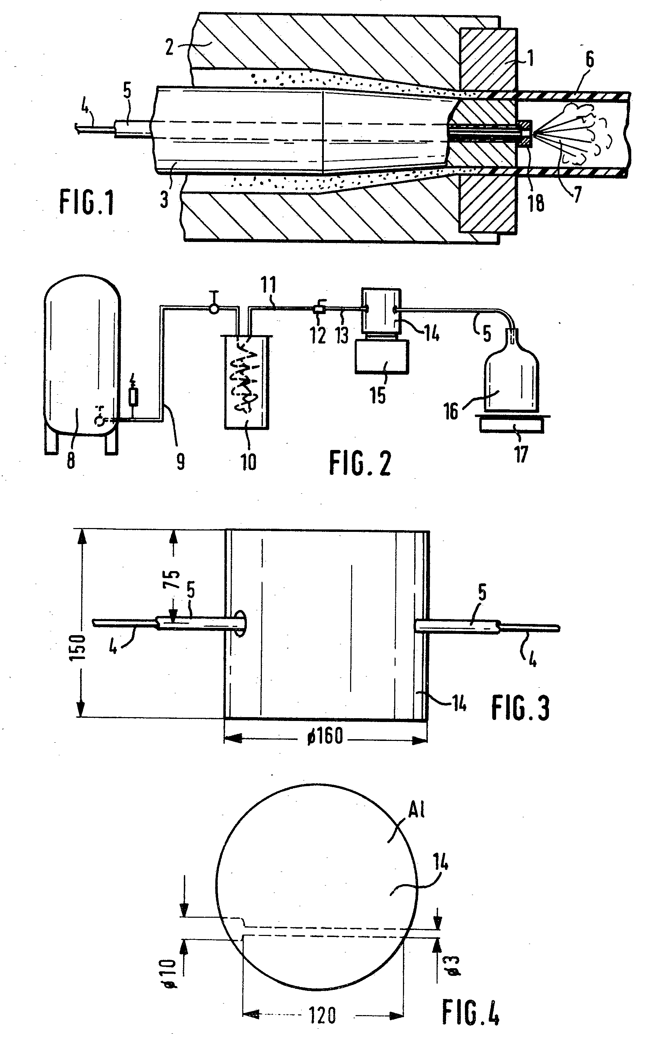

Die in Fig. 1 dargestellte Vorrichtung zeigt den Austrittsbereich eines Extrusionswerkzeuges, bestehend aus der Extrusionsdüse 1, dem Düsenhalter 2 und dem Dorn 3. Erfindungsgemäß befindet sich im Dorn 3 ein Kapillarschlauch 4 aus PTFE mit einem Innendurchmesser von 1 mm und einem Außendurchmesser von 2 mm. Der Kapillarschlauch 4 ist von einem Isolierschlauch 5 aus PTFE umgeben, der einen Innendurchmesser von 2,2 mm und einen Außendurchmesser von 2,8 mm besitzt. Die Bohrung zur Aufnahme des Kapillarschlauches 4 und Isolierschlauches 5 hat einen Durchmesser von 3 mm. Der als Kühlmittel verwendete unterkühlte flüssige Stickstoff 7 durchströmt den Kapillarschlauch 4 und gelangt weitgehend in flüssiger Form in das extrudierte Kunststoff-Hohlprofil 6. Am Austrittsende des Kapillarschlauches 4 kann eine dem Stickstoffdurchsatz angepaßte Düse 18 angebracht sein. Durch sie wird der flüssige Stickstoff zerstäubt, wodurch die Verdampfungsgeschwindigkeit und Kühlwirkung erhöht wird. Die Fig. 2 bis 4 zeigen eine Versuchsanordnung, mit der die Durchführbarkeit des erfindungsgemäßen Verfahrens unter verschiedenen Arbeitsbedingungen geprüft wurde.

Der flüssige Stickstoff wurde dem Standtank 8 entnommen und durch eine 10 m lange normalisierte Leitung 9 dem Unterkühler 10 zugeführt. Der Speicherdruck lag zwischen 3,3 und 5,3 bar. Die Wärme, die dem flüssigen Stickstoff beim Durchfluß durch die Leitung 9 zugeführt wurde, wurde ihm im Unterkühler 10 durch Wärmeaustausch mit verdampfendem flüssigem Stickstoff unter atmosphärischem Druck wieder entzogen. Für die Versuche stand somit unterkühlter flüssiger Stickstoff zur Verfügung, der über die isolierten PA-Schläuche 11 und 13 sowie den Kugelhahn 12 in den Aluminiumzylinder 14 geleitet wurde. Der Aluminiumzylinder 14 simulierte das Extrusionswerkzeug. Einzelheiten seiner Abmessungen sowie der Anordnung des Kapillarschlauches gemäß der Erfindung können den Fig. 3 und 4 entnommen werden.

Der Aluminiumzylinder 14 wurde mittels einer Heizplatte 15 auf die jeweils gewünschten Temperaturen erhitzt. Der flüssige Stickstoff wurde erfindungsgemäß mittels eines Kapillarschlauches 4, der in einem Isolierschlauch 5 eingezogen war, durch den Aluminiumzylinder 14 geführt und in das Dewar-Gefäß 16 geleitet. Der im Dewar-Gefäß 16 aufgefangene flüssige Stickstoff wurde mit der Tischwaage 17 gewogen.

Der Kapillarschlauch 4 hatte 1 mm Innendurchmesser und 2 mm Außendurchmesser. Es wurden Kapillarschläuche 4 aus PA-12, Polyimid und PTFE untersucht. Der Isolierschlauch 5 bestand aus PTFE und besaß einen Innendurchmesser von 2,2 mm und einen Außendurchmesser von 2,8 mm. Es wurden außerdem Vergleichsversuche mit einem Kapillarschlauch der Nennweite 2 mm durchgeführt.

Generell ergaben die Versuche folgendes:

Ohne vorherige Unterkühlung gelangt nur gasförmiger Stickstoff durch den Kapillarschlauch 4.

Ferner ist der Isolierschlauch 5 erforderlich, wenn ein ausreichender Durchsatz an flüssigem Stickstoff erreicht werden soll. PA ist als Material für den Kapillarschlauch 4 ungeeignet, da der Schlauch bei heißem Werkzeug und Unterbrechung der Stickstoffzufuhr schmilzt. Polyimid und PTFE werden daher als Materialien für den Kapillarschlauch 4 bevorzugt.

Die Versuchsergebnisse sind in der folgenden Tabelle zusammengefaßt:

| Schlauch (Innen⌀) | Temperatur (°C) | Druck (bar) | Durchsatz (kg/h) |
|---|---|---|---|
| 2 mm | +20 | 5 | 131 |
| 2 mm | +20 | 3,5 | 111 |
| 1 mm | +20 | 5,3 | 30,6 |
| 1 mm | +20 | 4,7 | 26,7 |
| 1 mm | +100 | 4,4 | 18,5 |
| 1 mm | +150 | 4,3 | 17,5 |
| 1 mm | +200 | 3,3 | 14,25 |

Aus der Tabelle ist zu erkennen, daß der Flüssig-Stickstoff-Durchsatz durch das warme Werkzeug gegenüber dem Durchsatz durch das kalte Werkzeug zwar geringer ist, aber der größte Teil dennoch in flüssiger Form in das Dewar-Gefäß 16 (bzw. in das Kunststoff-Hohlprofil 6) gelangt.

Die Werkzeugtemperatur hat überraschenderweise keinen großen Einfluß auf die Durchsatzmenge. Durch einen Kapillarschlauch mit 1 mm Nennweite lassen sich je nach Vordruck (3—6 bar) 12 bis 23 kg $LN_2$/h durchsetzen. Die Mengenregelung kann demnach über den Druck oder ein Feinstregelventil erfolgen.

Im praktischen Betrieb wurde bei einem Hohlprofil mit einem spez. Gewicht von 360 g/m durch das erfindungsgemäße Verfahren die stündliche Extruderleistung um 20% gesteigert. Der spezifische Stickstoffverbrauch beträgt hierbei 0,2 kg/kg Produkt.

**Patentanspruch**

Verfahren zur Kühlung von Kunststoff-Hohlprofilen beim Extrudieren durch direkten Wärmeaustausch mit flüssigem Stickstoff, der durch eine Bohrung im Dorn (3) des Extrusionswerkzeuges in das Innere des Kunststoff-Hohlprofils (6) eingeführt wird, dadurch gekennzeichnet, daß der Stickstoff unter einem Druck von 2 bis 8 bar steht und eine Temperatur unterhalb seines Siedepunktes besitzt, also unterkühlt ist, und daß der Stickstoff im Extrusionswerkzeug durch einen von einem Isolierschlauch (5) umgebenen Kapillarschlauch (4) mit einem Innendurchmesser bis zu 1,2 mm strömt.

**Claim**

Process for cooling of hollow synthetic material profiles during extrusion by means of direct heat exchange with liquid nitrogen, which is introduced via a boring in the mandrel (3) of the extrusion tool into the interior of the hollow synthetic material (6), characterised in that the nitrogen has a pressure of 2 to 8 bar and a temperature below its boiling point, what means that it is subcooled, and that the liquid in the extrusion tool moves through a capillary tube (4) with a maximum inside diameter of 1,2 mm, which capillary tube is enclosed by an insulation tube (5).

**Revendication**

Procédé pour refroidir des profilés creux en matière synthétique lors de l'extrusion par échange direct de chaleur avec de l'azote liquide, qui est introduit à l'intérieur du profilé creux en matière synthétique (6) à travers un perçage de la broche (3) de l'outil d'extrusion, caractérisé en ce que, l'azote liquide est à une pression de 2 à 8 bars et à une température en-dessous de son point d'ébullition, c'est-à-dire est sous-refroidi, et en ce que l'azote s'écoule dans l'outil d'extrusion à travers un tuyau capillaire (4) ayant un diamètre intérieur allant jusqu'à 1,2 mm et entouré d'un tuyau isolant.

FIG.1

FIG.2

FIG.3

FIG.4